# EUROPEAN PATENT SPECIFICATION

(11) **EP 0 470 767 B1**
(45) Date of publication and mention of the grant of the patent: **06.11.1996**
(21) Application number: 91307073.6
(22) Date of filing: 01.08.1991
(51) Int. Cl.: A47C 27/00

(54) **Massaging mattresses or pillows**
Massagematratze oder Massagekopfkissen
Matelas ou oreiller de massage

(30) Priority: 04.08.1990 CN 90217459; 28.12.1990 CN 90110287
(43) Date of publication of application: 12.02.1992
(73) Proprietor: Chan, Hoi Chau, Baoan County, Shenzhen (CN)
(72) Inventor: Chan, Hoi Chau, Baoan County, Shenzhen (CN)
(74) Representative: Newby, John Ross

(56) References cited:
- GB-A- 2 224 434
- JP-A-61 073 306

## Description

The invention relates to personal and domestic articles, particularly to mattresses and pillows which have a massaging function.

Prior art mattresses conventionally comprise a resilient interior of, for example, fibre, e.g. coconut fibre, foam, interior springing, with a cover thereover. Although this kind of mattress is generally comfortable for sleep, it has little or no therapeutic function, and tends to be uncomfortable in hot summer weather. Chinese Patent CN2032055U discloses a kind of magnetic-therapeutic spring mattress, in which there are provided magnetic therapeutic lumps with various field intensities arranged according to the degree of magnetic endurance of different parts of the human body. However the therapeutic function of the mattress described in Chinese Patent CN2032055U only affects a person's neck and waist, and no ventilation is provided, thus making the mattress unsuitable for use in hot summer weather. Another Chinese Patent CN2045599U discloses a mattress which has ventilation and cooling functions and CN2044145U discloses a mattress suitable for use in both summer and winter. Although both these kinds of mattresses are suitable for summer use, neither of them has any magnetic-therapeutic function and their structures are relatively complex.

GB-A-2,224,434, the source of the preamble of claim 1, discloses a number of mattresses having arrays of beads designed to apply pressure to specific body points of a person lying on the mattress. In one embodiment, disk-shaped magnets are also incorporated into the mattress for the purpose of supplying theraputic magnetic fluxes. The disks are oriented with their flat surfaces parallel to the surface of the mattress and are positioned below the support surfaces of the beads; they play no part in supporting or massaging the body of the person lying on the mattress.

According to the present invention, there is provided a mattress or pillow comprising a compressible interior and supported thereby an array of non-resilient beads of barrel shape and of magnets, characterised in that each magnet is of at least generally cylindrical shape. Conveniently the interior and array are overlain by a flexible covering (e.g. of gauze and/or cloth).

The mattress or pillow according to the present invention can be of simple structure and has magnetic therapeutic and therapeutic massage functions. Also ventilation is preferably provided, making the mattress or pillow suitable and comfortable for use in summer.

Preferably the compressible interior is in the form of a resilient interior. For example the resilient interior may be of a plastics foam material, a sprung interior or even a liquid-filled or gas-filled, e.g. air-filled, interior.

Suitably the beads and magnets in the array are interconnected by one or more cords threaded therethrough to form an interconnected layer, which layer can be adhered to the compressible interior. The arrangement of the beads and magnets in the array, in particular the number of beads provided between magnets, is chosen according to the desired effect.

The beads used are most suitably wooden beads although beads of plastics or other materials could be used.

Preferred embodiments of the invention are defined in the dependent claims.

Embodiments of the invention will now be described, by way of example, with reference to the accompanying drawings in which:
Figure 1 is sectional view of a mattress according to a first embodiment of the invention;
Figure 2 is a plan of the array of wooden beads and magnets of the embodiment of Figure 1;
Figure 3 is a sectional view of a mattress according to a second embodiment of the invention, in which the traditional combination of spring coil matrix and plastics foam are used as a resilient mattress interior;
Figure 4 is a detailed drawing of a wooden bead used according to the present invention;
Figure 5 is a detailed drawing of a magnet used according to the present invention; and
Figure 6 is a sectional view of a pillow having a curved surface according to a third embodiment of the invention.

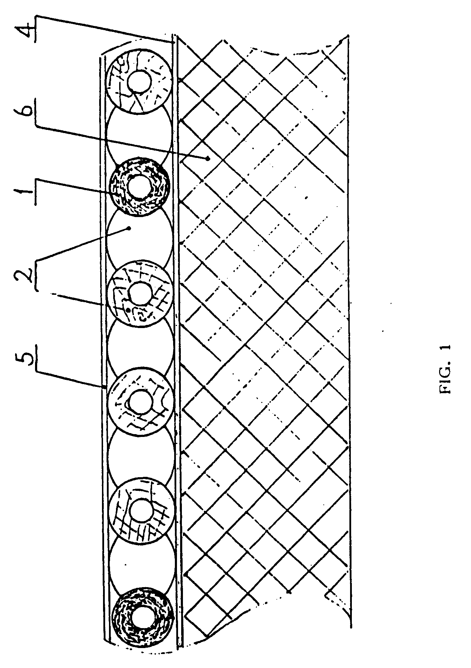

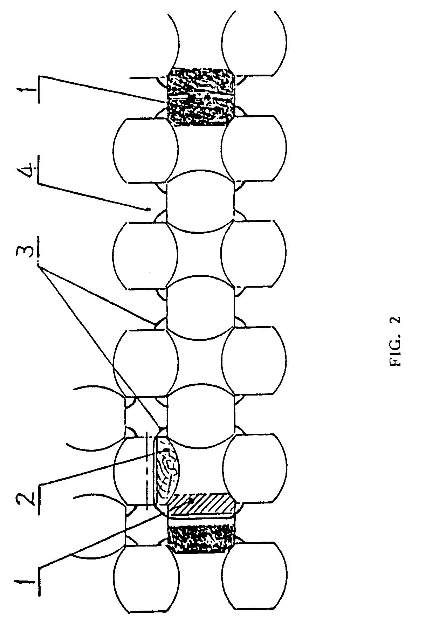

With reference to Figures 1 and 2 of the accompanying drawings, the mattress comprises a resilient interior 6 and a covering 5, between which is an array of wooden beads 2 and magnets 1. The array of wooden beads 2 and magnets 1 is connected by cords 3 to form a network layer, which is adhered to the resilient interior 6 by means of glue 4.

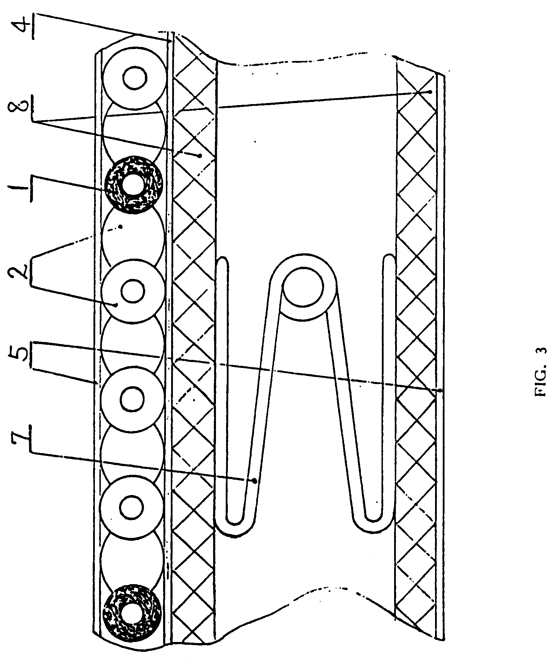

The resilient interior 6 can be entirely made of a sponge or of plastics foam 8, or of a combination of spring matrix 7, e.g. coil spring matrix, and plastics foam 8 as shown in Figure 3. Alternatively it is possible for the interior of the mattress to be in the form of an air-filled or liquid-filled inner mattress.

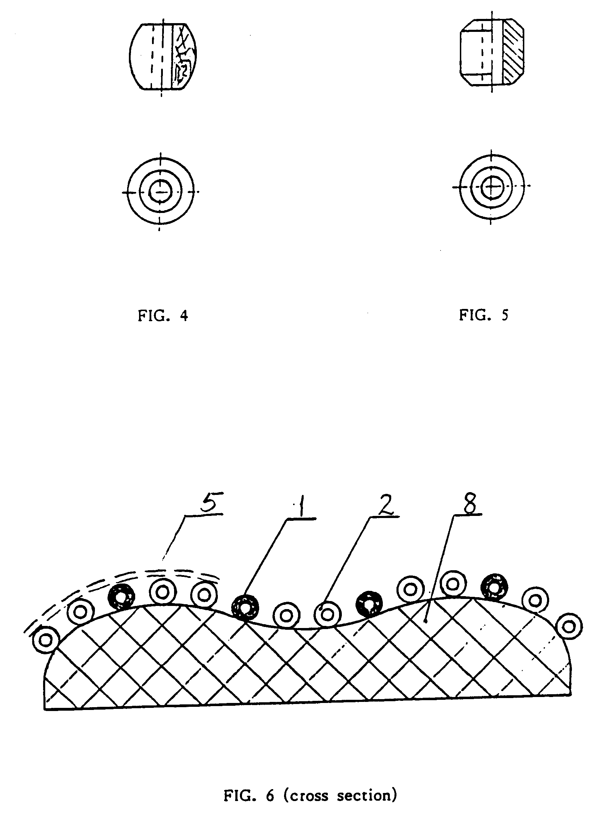

As shown in Figure 4, the wooden beads are of substantially barrel shape, the maximum diameter and the axial length of the beads being approximately the same, typically approximately 16 mm. Each bead has an axially extending hole through its centre. The beads can be made of hard woods, such as maple wood, mulberry wood or northeast China ash wood.

As shown in Figure 5, the magnets are of generally cylindrical form with bevelled ends with the cylinder diameter and axial length of the magnet being substantially the same, typically approximately 16 mm. Also each magnet has an axially extending hole through its centre. The magnet can be made of permanently magnetic ferrite, alnico, permanently magnetic rare-earths-cobalt or a processable permanent magnet and has a surface field-intensity of from 500 to 2,000 Gauss.

The cord 3 can be of nylon, polyamide fibre, waxed cotton thread or silk thread.

The glue 4 can suitably be a glue conventionally used for indoor decoration.

With reference to Figure 6, there is shown a pillow comprising a resilient interior having a curved surface and an array of wooden beads 2 and magnets 1 such that two wooden beads may be set between two magnets. The pillow can include a covering (partially shown in dashed lines at 5) which can comprise a layer of gauze of relatively large mesh and a layer of cotton cloth wrapped over the gauze.

In a mattress according to the invention, the array of wooden beads 2 and magnets 1, which array is near the surface layer of the mattress, is suitably such that there is provided one magnet every three to every five wooden beads apart, and that the distance between the magnets is from 7 to 12 cm. The magnets and the wooden beads are strung together by means of cords to form a large network whose dimensions meets the mattress standard, and which can bear 600 kg pressure without getting loose. Each wooden bead has a curved surface so that a certain amount of air is held in the crevices between them. When the mattress is pressed or moved slightly, air will flow through these crevices and the beads and magnets themselves will also roll slightly, so as to give anyone lying on the mattress the feeling of being massaged by hands. In addition to the massaging therapy function, the magnetic field of the magnets will also affect the body while the body touches the mattress so that waist pain and back soreness may be relieved and blood circulation facilitated and metabolism promoted, in this way to assist relaxation of weary muscles. Since wooden beads and magnets in the array have curved surfaces to permit air flow, the mattress according to the invention is not hot to lie on even in hot summer weather. Further the mattress is believed to have certain curing effects over rheumatism and skin diseases.

Compared with various famous brand mattresses now available in the market, the mattress according to the present invention is not only an article for daily use but also provides medical treatment. Also, it can be used comfortably not only in autumn, winter and spring, but also in hot summer in places where no air conditioning is available, and is comfortable for all types of rest. Further the mattress according to the invention can be suitable for rolling into a bundle and to be tied up, or spread on an uneven surface without damaging its function. It is therefore convenient for storage or transport, can be nearly 3\4 lighter in weight than other products, and very suitable for travel purposes. Moreover, manufacturing processes and techniques required according to the invention may be simple, and thus manufacturing costs may be reduced.

After being used by certain group of individuals including medical personnel, it was considered that the mattress and pillow according to the invention not only make sleeping comfortable and increase energy after the sleep, but also lower the blood pressure, release pain, diminish inflammation, quieten the nervous system, cure rheumatism and promote body health.

Although the invention has been primarily described in relation to a mattress having a resilient interior, the invention may also be applied to pillows having resilient interiors or to pillows having interiors which, although compressible, may not be truly resilient, e.g. pillows filled with feathers or the like.

Although the array of beads and magnets are primarily intended to be incorporated within a mattress or pillow at the manufacturing stage, it will be appreciated that a flexible array of linked together beads and magnets could be positioned on top of an existing mattress or pillow. In this event the array could be covered by a sheet or pillow case or the like. The array could be allowed to rest on top of the mattress or pillow or could be secured, e.g. tied, thereto.

## Claims

1. A mattress or pillow comprising a compressible interior (6) and supported thereby an array of non-resilient beads (2) of barrel shape and of magnets (1), characterised in that each magnet (1) is of at least generally cylindrical shape.

2. A mattress or pillow according to claim 1, characterised in that the magnets (1) are of permanently magnetic ferrite, alnico, permanently magnetic rare-earth-cobalt or processable permanent magnet material.

3. A mattress or pillow according to claim 1 or claim 2, characterized in that the magnets (1) have a surface field-intensity from 500 to 2,000 Gauss.

4. A mattress or pillow according to any preceding claim, characterised in that the beads (2) and magnets (1) in the array are interconnected by cord (3) to form a layer, which layer is adhered to the compressible interior (6).

5. A mattress or pillow according to any preceding claim, characterised in that the maximum diameter of each bead (2) and the axial length of each bead are approximately the same, and each bead has an axially extending hole through its centre.

6. A mattress or pillow according to any preceding claim, characterised in that the beads (2) are of wood, such as maple wood, mulberry wood or northeast China ash wood.

7. A mattress or pillow according to any preceding claim, characterised in that each magnet (1) has an approximately equal diameter and axial length and has an axially extending hole through its centre.

8. A mattress or pillow according to claim 4, characterised in that the cord (3) is of nylon, polyamide fibre, waxed cotton thread or silk thread.

9. A mattress or pillow according to any preceding claim, characterised in that said compressible interior (6) is resilient.

10. A mattress or pillow according to any preceding claim, characterised in that a flexible covering (5) is provided over the compressible interior (6) and array of beads (2) and magnets (1).

11. A mattress according to any preceding claim, characterised in that in the array there is provided one magnet (1) every three to every five beads (2) and the distance between the magnets (1) is from a few to some dozen centimetres.

12. A mattress according to any preceding claim, which comprises a resilient interior (6), characterised in that the interior (6) is a combination of a spring matrix (7) and foam (8).

13. A pillow according to any one of claims 1 to 10, characterised in that it has a curved surface and two beads (2) are provided between each magnet (1) pair.

## Patentansprüche

1. Matratze oder Kissen mit einem zusammendrückbaren Kern (6) und einer davon gestützten Anordnung von nicht elastischen tonnenförmigen Wülsten (2) und von Magneten (1), dadurch gekennzeichnet, daß jeder Magnet (1) zumindest allgemein zylindrischer Form ist.

2. Matratze oder Kissen nach Anspruch 1, dadurch gekennzeichnet, daß die Magnete (1) aus dauermagnetischem Ferrit, Alnico, dauermagnetischem Seltenerdenkobalt oder verarbeitbarem, dauermagnetischem Material bestehen.

3. Matratze oder Kissen nach Anspruch 1 oder 2, dadurch gekennzeichnet, daß die Magnete (1) eine Flächenfeldstärke von 500 bis 2000 Gauß aufweisen.

4. Matratze oder Kissen nach einem der vorhergehenden Ansprüche, dadurch gekennzeichnet, daß die Wülste (2) und Magnete (1) in der Anordnung zur Bildung einer Schicht durch Schnüre (3) verbunden sind, wobei die Schicht an den zusammendrückbaren Kern (6) geklebt ist.

5. Matratze oder Kissen nach einem der vorhergehenden Ansprüche, dadurch gekennzeichnet, daß der maximale Durchmesser jeder Wulst (2) und die axiale Länge jeder Wulst ungefähr gleich sind und jede Wulst ein axial durch ihre Mitte verlaufendes Loch aufweist.

6. Matratze oder Kissen nach einem der vorhergehenden Ansprüche, dadurch gekennzeichnet, daß die Wülste (2) aus Holz wie zum Beispiel Ahornholz, Maulbeerbaumholz oder nordostchinesischem Eschenholz bestehen.

7. Matratze oder Kissen nach einem der vorhergehenden Ansprüche, dadurch gekennzeichnet, daß jeder Magnet (1) ungefähr den gleichen Durchmesser und die gleiche axiale Länge sowie ein axial durch seine Mitte verlaufendes Loch aufweist.

8. Matratze oder Kissen nach einem der vorhergehenden Ansprüche, dadurch gekennzeichnet, daß die Schnur (3) aus Nylon, Polyamidfaser, Wachsbaumwolldraht oder einem Seidenfaden besteht.

9. Matratze oder Kissen nach einem der vorhergehenden Ansprüche, dadurch gekennzeichnet, daß der zusammendrückbare Kern (6) elastisch ist.

10. Matratze oder Kissen nach einem der vorhergehenden Ansprüche, dadurch gekennzeichnet, daß über dem zusammendrückbaren Kern (6) und der Anordnung aus Wülsten (2) und Magneten (1) ein flexibler Überzug (5) vorgesehen ist.

11. Matratze nach einem der vorhergehenden Ansprüche, dadurch gekennzeichnet, daß in der Anordnung alle drei bis fünf Wülste (2) ein Magnet (1) vorgesehen ist und der Abstand zwischen den Magneten (1) zwischen ein paar Zentimetern und ungefähr einem Dutzend Zentimeter beträgt.

12. Matratze nach einem der vorhergehenden Ansprüche, die einen elastischen Kern (6) enthält, dadurch gekennzeichnet, daß der Kern (6) eine Kombination aus Federmatratze (7) und Schaumstoff (8) ist.

13. Kissen nach einem der Ansprüche 1 bis 10, dadurch gekennzeichnet, daß es eine gekrümmte Fläche hat und zwischen jedem Paar Magnete (1) zwei Wülste (2) vorgesehen sind.

## Revendications

1. Matelas ou oreiller comprenant un intérieur compressible (6) et, supporté par celui-ci, un réseau de perles non-élastiques (2) en forme de tonneau et d'aimants (1), caractérisés en ce que chaque aimant (1) est de forme au moins généralement cylindrique.

2. Matelas ou oreiller selon la revendication 1, caractérisés en ce que les aimants (1) sont en ferrite magnétique permanent, alnico, cobalt des terres rares magnétique permanent ou matériau magnétique permanent traitable industriellement.

3. Matelas ou oreiller selon la revendication 1 ou la revendication 2, caractérisés en ce que les aimants (1) ont une intensité de champ surfacique comprise entre 500 et 2000 Gauss.

4. Matelas ou oreiller selon l'une quelconque des revendications précédentes, caractérisés en ce que les perles (2) et aimants (1) du réseau sont liés entre eux à l'aide d'une ficelle (3) pour former une couche, ladite couche étant collée à l'intérieur compressible (6).

5. Matelas ou oreiller selon l'une quelconque des revendications précédentes, caractérisés en ce que le diamètre maximal de chaque perle (2) et la longueur axiale de chaque perle sont approximativement les mêmes, et chaque perle est perforée le long de son axe central.

6. Matelas ou oreiller selon l'une quelconque des revendications précédentes, caractérisés en ce que les perles (2) sont en bois, tel que du bois d'érable, du bois de mûrier ou du bois d'orme de Chine.

7. Matelas ou oreiller selon l'une quelconque des revendications précédentes, caractérisés en ce que chaque aimant (1) a un diamètre et une longueur axiale approximativement égaux et est perforé le long de son axe central.

8. Matelas ou oreiller selon la revendication 4, caractérisés en ce que la ficelle (3) est en nylon, en fibre de polyamide, en fil de coton poissé ou en fil de soie.

9. Matelas ou oreiller selon l'une quelconque des revendications précédentes, caractérisés en ce que l'intérieur compressible (6) est élastique.

10. Matelas ou oreiller selon l'une quelconque des revendications précédentes, caractérisés en ce qu'un revêtement souple (5) est prévu sur l'intérieur compressible (6) et le réseau de perles (2) et d'aimants (1).

11. Matelas selon l'une quelconque des revendications précédentes, caractérisé en ce qu'à l'intérieur du réseau un aimant (1) est prévu toutes les trois à cinq perles (2) et la distance entre les aimants (1) est comprise entre quelques centimètres et environ une douzaine de centimètres.

12. Matelas selon l'une quelconque des revendications précédentes, comprenant un intérieur élastique (6), caractérisé en ce que l'intérieur (6) est une combinaison d'une matrice de ressorts (7) et de mousse (8).

13. Oreiller selon l'une quelconque des revendications 1 à 10, caractérisé en ce qu'il possède une surface courbe et deux perles (2) sont prévues entre chaque paire d'aimants (1).
